# EUROPEAN PATENT APPLICATION

(11) **EP 4 382 866 A1**
(43) Date of publication of application: **12.06.2024**
(21) Application number: 23199443.5
(22) Date of filing: 25.09.2023
(51) Int. Cl.: G01C 23/00, G08G 5/00

(54) **METHOD, SYSTEM, AND COMPUTER-READABLE MEDIUM FOR MONITORING AND PREDICTING GREENHOUSE GAS EMISSIONS FOR A FLIGHT OF AN AIRCRAFT**

(30) Priority: 05.12.2022 US 202218061845
(71) Applicant: The Boeing Company, Arlington, VA 22202 (US)
(72) Inventor: SANZONE, Andrea, Arlington, 22202 (US); SAHLE, Hilna, Arlington, 22202 (US)
(74) Representative: Boult Wade Tennant LLP

(57) **Abstract**

The present disclosure is directed to a method and system for monitoring and predicting greenhouse gas emissions for a flight of an aircraft. The system receives navigation and location data of a flight of an aircraft through one or more regions of travel such as an airspace. The system further obtains fuel consumption data of the flight of the aircraft through the one or more regions of travel. Using mathematical models, the system then determines the greenhouse gas emissions of the aircraft through the one or more regions of travel. The system then compares the greenhouse gas emissions of the aircraft with the navigation and location data to determine one or more correlations between the greenhouse gas emissions data and the one or more regions of travel. The system then outputs the correlations to various other systems (e.g., a display or machine learning system) for further analysis and processing.

## Description

### BACKGROUND

### Field of the Disclosure

The present disclosure is generally related to monitoring greenhouse gas emissions for aircraft. More particularly, the present disclosure is related to monitoring and predicting greenhouse gas emissions of an aircraft using a machine learning model.

### Description of Related Art

Fighting global climate change has become a focus of many companies and governmental organizations around the world as global average temperatures increase, extreme droughts and flooding become more prevalent, and greenhouse gas emissions continue to rise and contribute to these issues. In order to develop strategies to minimize greenhouse gas emissions, it would be advantageous to first have a system that accurately details greenhouse gas emissions of various vehicles, such as aircraft. Existing solutions to calculate aircraft emissions are very approximate and mostly make use of an estimated distance between two destinations (e.g., great circle distance) to calculate fuel burnt, and thereby the greenhouse gas emissions of a flight. Additionally, existing solutions do not provide specific emissions at regional and local levels such as at the airspace level.

It would therefore be desirable to have a system and method that takes into account at least some of the issues discussed above, as well as other possible issues.

### SUMMARY

Examples of the present disclosure are directed to a method, system, and computer-readable medium for monitoring and predicting greenhouse gas emissions for a flight of an aircraft. The system receives navigation and location data of a flight of an aircraft through one or more regions of travel such as an airspace. The system further obtains fuel consumption data of the flight of the aircraft through the one or more regions of travel. Using mathematical models, the system then determines the greenhouse gas emissions of the aircraft through the one or more regions of travel. The system then compares the greenhouse gas emissions of the aircraft with the navigation and location data to determine one or more correlations between the greenhouse gas emissions data and the one or more regions of travel. The system then outputs the correlations to various other systems (e.g., a display or machine learning system) for further analysis and processing. For example, the system transmits the correlations to a display for output to a user, or the system outputs the correlations to a machine learning model for predicting future greenhouse gas emissions for other aircraft that are flying through the one or more regions of travel.

The present disclosure thus includes, without limitation, the following examples.

Some examples provide a method for monitoring and predicting greenhouse gas emissions for a flight of an aircraft, the method comprising: accessing, using one or more processors in communication with a non-transitory computer-readable medium having executable instructions stored thereon, navigation and location data relating to the flight of the aircraft, including navigation and location data of the flight through one or more regions of travel; obtaining fuel consumption data of the flight of the aircraft through the one or more regions of travel; converting the fuel consumption data into greenhouse gas emissions data of the flight of the aircraft through the one or more regions of travel; comparing the greenhouse gas emissions data of the aircraft with the navigation and location data to determine one or more correlations between the greenhouse gas emissions data and the one or more regions of travel; and outputting the one or more correlations to a display for monitoring the greenhouse gas emissions of the aircraft.

Obtaining the fuel consumption data of the flight of the aircraft ay include calculating fuel consumption of the flight using mathematical models based on the location and navigation data, aircraft type and configuration, and weather data.

The aircraft type and configuration may include data regarding aircraft model or class, aircraft weight, a number of seats onboard and aircraft, winglet parameters, engine type, or a load factor of the aircraft.

The weather data may include data regarding wind intensity and direction, temperature, or humidity of surroundings of the aircraft during the flight.

Obtaining the fuel consumption data of the flight of the aircraft may include obtaining the fuel consumption data directly from the aircraft.

Obtaining the fuel consumption data of the flight of the aircraft may include obtaining flight parameter data of the flight; wherein converting the fuel consumption data into greenhouse gas emissions data includes applying a mathematical model to one or both of the fuel consumption data and the flight parameter data to obtain the greenhouse gas emissions data.

The mathematical model may be a linear relationship function and the greenhouse gas emissions data comprises carbon dioxide emissions data; or wherein the greenhouse gas emissions data comprises emissions data for greenhouse gasses other than carbon dioxide, the method may comprise applying the mathematical model to both the fuel consumption data and the flight parameter data, and the flight parameter data may include at least flight altitude data and climatic conditions surrounding the aircraft.

The method may further comprise: building a machine learning model for predicting greenhouse gas emissions for a current or future flight of the aircraft using a machine learning algorithm trained with the one or more correlations between the greenhouse gas emissions data and the one or more regions of travel, one or more correlations between the greenhouse gas emissions and weather data, and the navigation and location data; predicting the greenhouse gas emissions of the aircraft for the current or future flight of the aircraft for the region of travel using the machine learning model and based on the one or more correlations between the greenhouse gas emissions data and the one or more regions of travel; and outputting the prediction to the display for use by a user to make decisions about altering the current or future flight of the aircraft.

The one or more correlations may include correlations characterizing quantities of greenhouse gas emissions of the aircraft over a given area of the one or more regions of travel.

Some other examples provide a system for monitoring and predicting greenhouse gas emissions for a flight of an aircraft, the system comprising: one or more processors in communication with a non-transitory computer-readable medium having executable instructions stored thereon, wherein, upon execution of the executable instructions, the one or more processors are configured to: access navigation and location data relating to the flight of the aircraft, including navigation and location data of the flight through one or more regions of travel; obtain fuel consumption data of the flight of the aircraft through the one or more regions of travel; convert the fuel consumption data into greenhouse gas emissions data of the flight of the aircraft through the one or more regions of travel; compare the greenhouse gas emissions data of the aircraft with the navigation and location data to determine one or more correlations between the greenhouse gas emissions data and the one or more regions of travel; and output the one or more correlations to a display for monitoring the greenhouse gas emissions of the aircraft.

The one or more processors being configured to obtain the fuel consumption data of the flight of the aircraft may include the one or more processors being configured to calculate fuel consumption of the flight using mathematical models based on the location and navigation data, aircraft type and configuration, and weather data.

The aircraft type and configuration may include data regarding aircraft model or class, aircraft weight, a number of seats onboard and aircraft, winglet parameters, engine type, or a load factor of the aircraft.

The weather data may include data regarding wind intensity and direction, temperature, or humidity of surroundings of the aircraft during the flight.

The one or more processors being configured to obtain the fuel consumption data of the flight of the aircraft may include the one or more processors being configured to obtain the fuel consumption data directly from the aircraft.

The one or more processors may be further configured to obtain flight parameter data of the flight; wherein the one or more processors being configured to convert the fuel consumption data into greenhouse gas emissions data may include the one or more processors being configured to apply a mathematical model to one or both of the fuel consumption data and the flight parameter data to obtain the greenhouse gas emissions data.

The mathematical model may be a linear relationship function and the greenhouse gas emissions data may comprise carbon dioxide emissions data; or wherein the greenhouse gas emissions data may comprise emissions data for greenhouse gasses other than carbon dioxide, the one or more processors may be further configured to apply the mathematical model to both the fuel consumption data and the flight parameter data, and the flight parameter data may include at least flight altitude data and the climatic conditions surrounding the aircraft.

The system may further comprise the one or more processors being configured to: build a machine learning model for predicting greenhouse gas emissions for a current or future flight of the aircraft using a machine learning algorithm trained with the one or more correlations between the greenhouse gas emissions data and the one or more regions of travel, one or more correlations between the greenhouse gas emissions and weather data, and the navigation and location data; predict the greenhouse gas emissions of the aircraft for the current or future flight of the aircraft for the region of travel using the machine learning model and based on the one or more correlations between the greenhouse gas emissions data and the one or more regions of travel; and output the prediction to the display for use by a user to make decisions about altering the current or future flight of the aircraft.

The one or more correlations may include correlations characterizing quantities of greenhouse gas emissions of the aircraft over a given area of the one or more regions of travel.

Some other examples provide a computer-readable medium for monitoring and predicting greenhouse gas emissions for a flight of an aircraft, the computer-readable storage medium being non-transitory and having computer-readable program code stored therein that, in response to execution by processing circuitry of an apparatus, causes the apparatus to at least: access navigation and location data relating to the flight of the aircraft, including navigation and location data of the flight through one or more regions of travel; obtain fuel consumption data of the flight of the aircraft through the one or more regions of travel; convert the fuel consumption data into greenhouse gas emissions data of the flight of the aircraft through the one or more regions of travel; compare the greenhouse gas emissions data of the aircraft with the navigation and location data to determine one or more correlations between the greenhouse gas emissions data and the one or more regions of travel; and output the one or more correlations to a display for monitoring the greenhouse gas emissions of the aircraft.

The apparatus may be further configured to: build a machine learning model for predicting greenhouse gas emissions for a current or future flight of the aircraft using a machine learning algorithm trained with the one or more correlations between the greenhouse gas emissions data and the one or more regions of travel, one or more correlations between the greenhouse gas emissions and weather data, and the navigation and location data; predict the greenhouse gas emissions of the aircraft for the current or future flight of the aircraft for the region of travel using the machine learning model and based on the one or more correlations between the greenhouse gas emissions data and the one or more regions of travel; and output the prediction to the display for use by a user to make decisions about altering the current or future flight of the aircraft.

These and other features and advantages of the present disclosure will be apparent from a reading of the following detailed description together with the accompanying drawings, which are briefly described below. The present disclosure includes any combination of two, three, four or more features or elements set forth in this disclosure, regardless of whether such features or elements are expressly combined or otherwise recited in a specific example described herein. This disclosure is intended to be read holistically such that any separable features or elements of the disclosure, in any of its examples, should be viewed as combinable, unless the context of the disclosure clearly dictates otherwise.

It will therefore be appreciated that this Brief Summary is provided merely for purposes of summarizing some examples so as to provide a basic understanding of some features of the disclosure. Accordingly, it will be appreciated that the above described examples are merely examples. Other examples and advantages will become apparent from the following detailed description taken in conjunction with the accompanying drawings which illustrate, by way of example, the principles of some described examples.

### BRIEF DESCRIPTION OF THE SEVERAL VIEWS OF THE DRAWING(S)

Having thus described the disclosure in general terms, reference will now be made to the accompanying drawings, which are not necessarily drawn to scale, and wherein:
FIG. 1 illustrates an example greenhouse gas emissions scenario;
FIG. 2A illustrates a block diagram of an example system for monitoring and predicting greenhouse gas emissions for a flight of an aircraft;
FIG. 2B illustrates a block diagram of an example greenhouse gas emissions prediction system;
FIG. 3 illustrates an example communications environment;
FIG. 4 illustrates a flow chart detailing steps of an example method; and
FIG. 5 illustrates an example apparatus for performing the method and other functions described herein.

### DETAILED DESCRIPTION OF THE DISCLOSURE

Some examples of the present disclosure will now be described more fully hereinafter with reference to the accompanying drawings, in which some, but not all features of the disclosure are shown. Indeed, various examples of the disclosure are embodied in many different forms and should not be construed as limited to the examples set forth herein; rather, these examples are provided so that this disclosure will be thorough and complete, and will fully convey the scope of the disclosure to those skilled in the art. For example, unless otherwise indicated, reference to something as being a first, second or the like should not be construed to imply a particular order. Also, something described as being above something else (unless otherwise indicated) is instead below, and vice versa; and similarly, something described as being to the left of something else is instead to the right, and vice versa. Like reference numerals refer to like elements throughout.

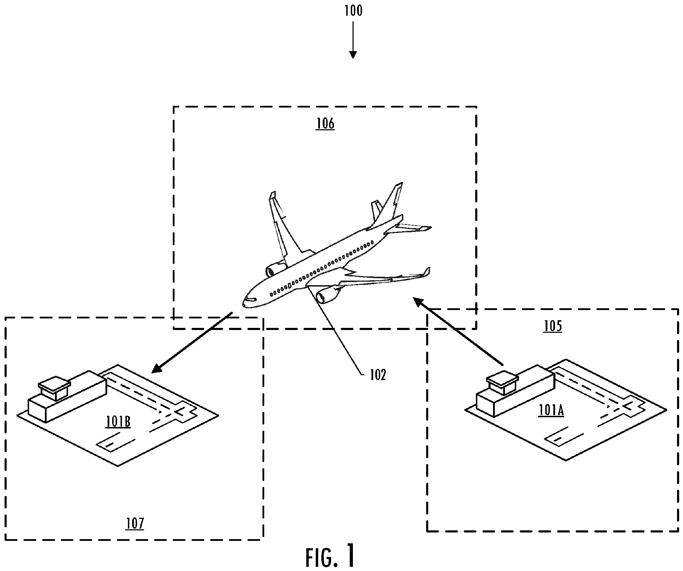

FIG. 1 illustrates an example greenhouse gas emission scenario 100 where an aircraft 102 is flying between a departure airport 101A and an arrival airport 101B. From the moment the engines of the aircraft are started, through takeoff, mid-flight, and during landing, the aircraft bums fuel, and consequently greenhouse gasses such as carbon dioxide are produced during combustion. However, at each phase of flight, such as at first region of travel 105 (e.g., the departure airport and airspace around it, the airspace being any portion of the atmosphere around the airport through which aircraft can fly), second region of travel 106 (e.g., the airspace where the aircraft is flying), and third region of travel 107 (e.g., the arrival airport and airspace around it), the aircraft burns different amounts of fuel and therefore produces different amounts of greenhouse gasses. Some non-carbon dioxide emissions are not linearly dependent on fuel consumption and also depend on other factors such as flight altitude and climatic conditions.

As described herein, the system, method, and computer-readable medium of the present disclosure are configured to monitor and predict greenhouse gas emissions based on different fuel consumption in these various regions of travel. Additionally, as described above, some non-carbon dioxide emissions depend also on factors other than fuel consumption only. These factors include altitude of the flight and weather conditions. By splitting the phase of the flight into multiple areas, a more accurate model of future greenhouse gas emissions is generated.

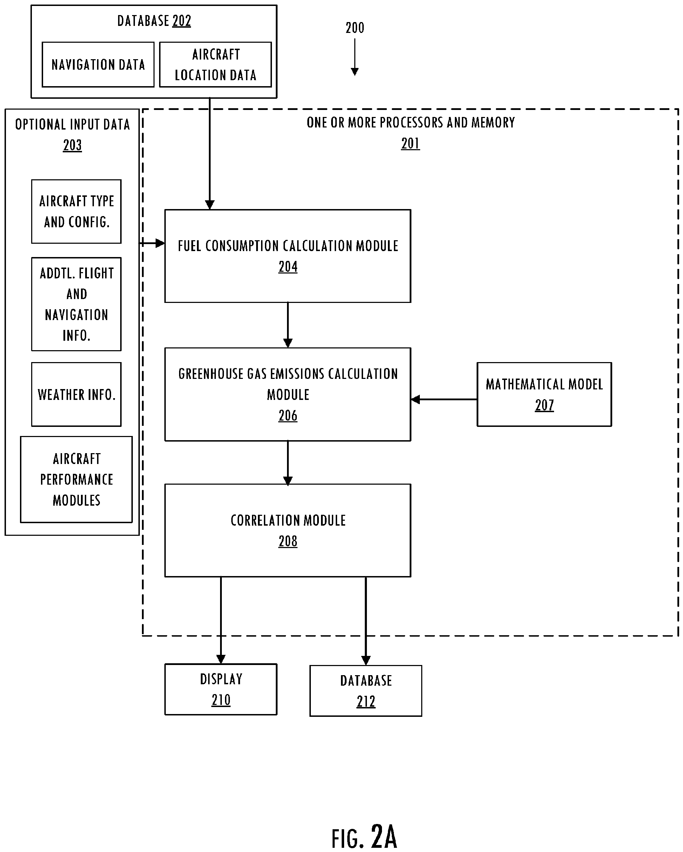

FIG. 2A illustrates a block diagram of a system 200 for monitoring and predicting greenhouse gas emissions for a flight of an aircraft, such as aircraft 102 shown in FIG. 1. In some examples, the system comprises one or more processors 201 in communication with a non-transitory computer-readable medium (e.g., memory) having executable instructions stored thereon. Upon execution of the executable instructions, the one or more processors are configured to perform various operations as described herein. In some examples, the system includes a fuel consumption calculation module 204 configured to receive or access input data such as navigation and location data relating to the flight of the aircraft, including navigation and location data of the flight through one or more regions of travel. That is, the system receives or accesses the navigation and location data of the aircraft through an region of travel such as first region of travel 105 or second region of travel 106 illustrated in FIG 1, for example. The navigation and location data are stored in any suitable data store or database 202 that the one or more processors is in communication with.

In some examples, the aircraft location data includes position, direction, altitude, origin-destination, and is obtained from any suitable system such as an Automatic Dependent Surveillance - Broadcast (ADS-B) data stream, and/or a multilateration system and/or a tablet PC (e.g., iPad) in the cockpit, etc. Tracking the position of an aircraft will give a more accurate estimation of the distance flown. In some examples, navigation data of the aircraft includes a 3D virtual representation of different airspaces (e.g., first region of travel 105 or third region of travel 107, etc.), airport details, waypoints, communications, and the like.

In some examples, the fuel consumption calculation module 204 receives optional input data 203 such as aircraft type and configuration (e.g., a Boeing 747, 737, etc., and number of seats on board or other configuration), additional flight and navigation information, weather information (e.g., from a satellite, radar, or weather station), or aircraft performance model (e.g., a mathematical model that provides accurate modeling of aircraft performance, including various performance behavior parameters, over the complete flight envelope; aircraft performance models are used, for example, to estimate thrust generated by the aircraft and fuel flow ). In some examples, aircraft type and configuration includes data regarding aircraft model or class, aircraft weight, a number of seats onboard and aircraft, winglet parameters, engine type, or a load factor of the aircraft. In some examples, the weather information or data includes data regarding wind intensity and direction, temperature, or humidity of surroundings of the aircraft during the flight.

Next, in some examples, the fuel consumption calculation module 204 is configured to obtain fuel consumption data of the flight of the aircraft through the one or more regions of travel. For example, the fuel consumption calculation module receives fuel consumption data directly from the aircraft (e.g., from the aircraft instruments detailing the fuel consumption of the aircraft while taking off and flying through the first region of travel 105 or any of the other regions of travel). In another example, the fuel consumption calculation module calculates the fuel consumption of the aircraft based on the other data received above. In some examples, the one or more processors 201 are configured to calculate fuel consumption of the aircraft during the flight using mathematical models based on the location and navigation data, aircraft type and configuration, and weather data. For example, the fuel consumption calculation module is configured to calculate fuel consumption based on the distance flown by the aircraft using mathematical models that, in some instances, require additional data sources as inputs to increase the accuracy of the calculation. This additional data includes the optional input data 203 described above, including aircraft information (e.g., type, configuration [such as seats and winglets], engine type, load factor, and the like), additional flight and navigation information (e.g., flight paths, flight phase, flight route, and the like), weather information (e.g., wind intensity and direction, temperature, humidity, and the like), aircraft performance models, and other data.

Once the system 200 obtains (i.e., determines, receives, etc.) the fuel consumption data, a greenhouse gas emissions calculation module 206 is configured to convert the fuel consumption data into greenhouse gas emissions data of the flight of the aircraft through the one or more regions of travel. In some examples, the greenhouse gas emissions calculation module is configured to apply a mathematical model 207 to the fuel consumption data to obtain the greenhouse gas emissions data. In some examples, the mathematical model is a linear relationship function and the greenhouse gas emissions data comprises carbon dioxide emissions data. In some examples, the linear relationship function includes a function that multiplies the number of kilograms of fuel burned by the aircraft by 3.16 (or approximately 3.16) to estimate the amount of carbon dioxide emitted by the aircraft. For example, if 500 kilograms of fuel are burned by the aircraft flying through the second region of travel 106 (e.g., during the main portion of the flight), about 1580 kilograms of carbon dioxide will be generated (i.e., 3.16 x 500 = 1580). In some other examples, the greenhouse gas emissions data comprise methane emissions data, water vapor emissions data, nitrous oxide emissions data, or ozone emissions data.

Next, in some examples, the system 200 further comprises a correlation module 208 configured to compare the greenhouse gas emissions data of the aircraft with the navigation and location data to determine one or more correlations between the greenhouse gas emissions data and the one or more regions of travel. In some examples, the one or more correlations include correlations characterizing quantities of greenhouse gas emissions of the aircraft over a given area of the one or more regions of travel, a given region, route, altitude, phase of flight, or other suitable area. For example, the correlation module determines an amount of greenhouse gas emitted during the first region of travel 105, the second region of travel 106, and/or the third region of travel 107 and produces correlations characterizing quantities of greenhouse gas emissions of the aircraft over the first region of travel, second region of travel, or third region of travel. In some examples, the correlation (or one or more correlations) is output to a display 210 for monitoring the greenhouse gas emissions of the aircraft. In some other examples, the correlation(s) is output to a database 212 for storage, and, as described herein, used for training a machine learning model.

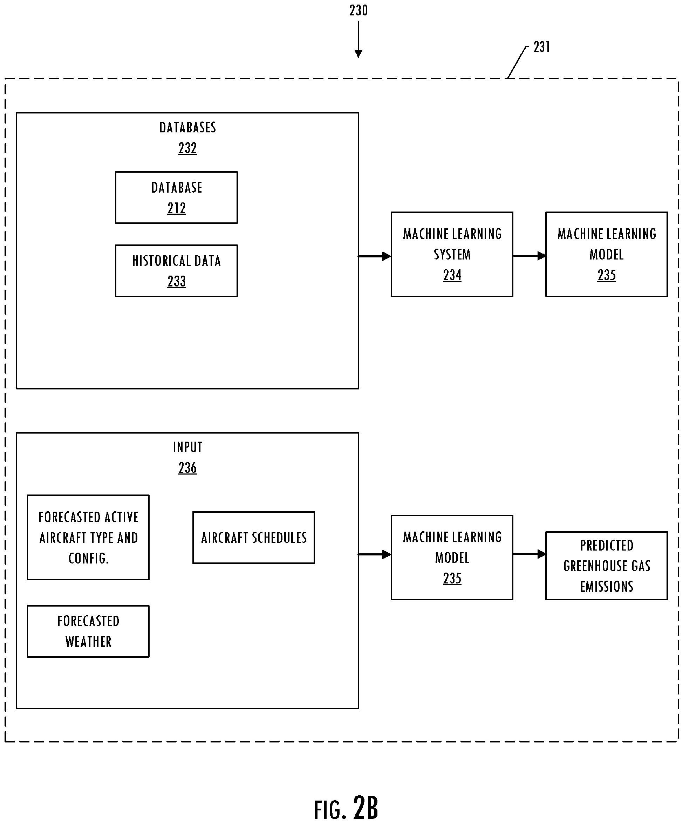

FIG. 2B illustrates an example prediction environment 230 controlled or generated by the system 200 from FIG. 2A. The prediction environment includes a prediction system 231 that includes one or more databases 232 such as the database 212 storing the correlations described above. The one or more databases further comprises historical data 233 (e.g., historical information on weather, historical aircraft information on active aircraft type and configuration, historical flight and navigation information, and any other suitable historical data). In some examples, the correlations from the database 212 and historical data are input into a machine learning system 234 including a machine learning algorithm.

In some examples, the one or more processors 201 of the system 200 are configured to build a machine learning model 235 for predicting greenhouse gas emissions for a current or future flight of the aircraft using the machine learning algorithm (e.g., of the machine learning system 234) trained with the correlations between the greenhouse gas emissions data and the one or more regions of travel, correlations between the greenhouse gas emissions and weather data, and the navigation and location data. In some examples, the prediction system includes the machine learning model 235, trained as described above, being fed various inputs 236 to form a prediction of the greenhouse gas emissions of a particular flight through a particular region of travel. For example, the machine learning model is provided with data characterizing a forecasted active aircraft type and configuration, forecasted weather, and aircraft schedules for a particular future flight of an aircraft over one or more regions of travel. The one or more processors of the system are configured to input the data above into the machine learning model and then predict the greenhouse gas emissions of the aircraft for the current or future flight of the aircraft for the region of travel using the machine learning model and based on the correlations between the greenhouse gas emissions data and the one or more regions of travel.

Once the prediction is made, the one or more processors are configured to output the prediction to the display 210 for use by a user to make decisions about altering the current or future flight of the aircraft. In other examples, the prediction is output to a memory for storing. In this and some other examples, the one or more processors are configured to output the prediction from the memory to a guidance system of the aircraft for the guidance system to automatically maneuver the aircraft through the regions of travel to minimize greenhouse gas emissions of the aircraft.

In some examples, the machine learning model 235 is a neural network and uses back propagation to iteratively refine weights and/or biases of the network. For example, actual greenhouse gas emissions data is compared against predicted emissions from the machine learning model and the comparison is used to fine tune/retrain the model. The fine tuning/retraining process is performed periodically, such as weekly or monthly. The machine learning algorithm is then redeployed after being retrained. In some examples, different machine learning models are built for each different type of aircraft or for each airspace or geographic regions. The retraining process is stopped once the difference between the predicted greenhouse gas emissions and actual greenhouse gas emissions reaches or goes below a predetermined threshold.

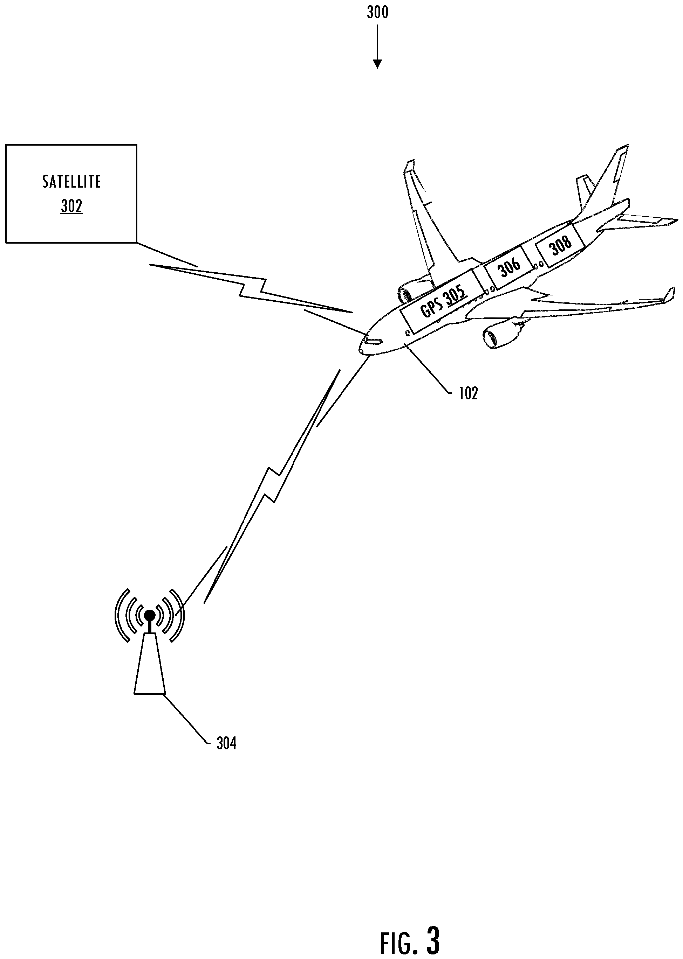

FIG. 3 illustrates an example communication environment 300 for how the location and navigation data of the aircraft 102 is collected. In some examples, the communication environment includes the aircraft, a satellite 302, and a ground receiver 304. A global positioning system (GPS) 305 on the aircraft communicates with the satellite that transmits positioning, navigation, and timing data. A navigation processor 306 on the aircraft determines the aircraft location data based on the positioning, navigation, and timing data from the satellite and sends data indicating the aircraft location to a transponder 308 on the aircraft. The transponder is configured to periodically broadcast wireless data streams that indicates the aircraft location to the ground receiver where the data is stored in one or more databases, such as database 202 shown in FIG. 2A, for use in monitoring the greenhouse gas emissions of the aircraft, developing the correlations described above, and make predictions.

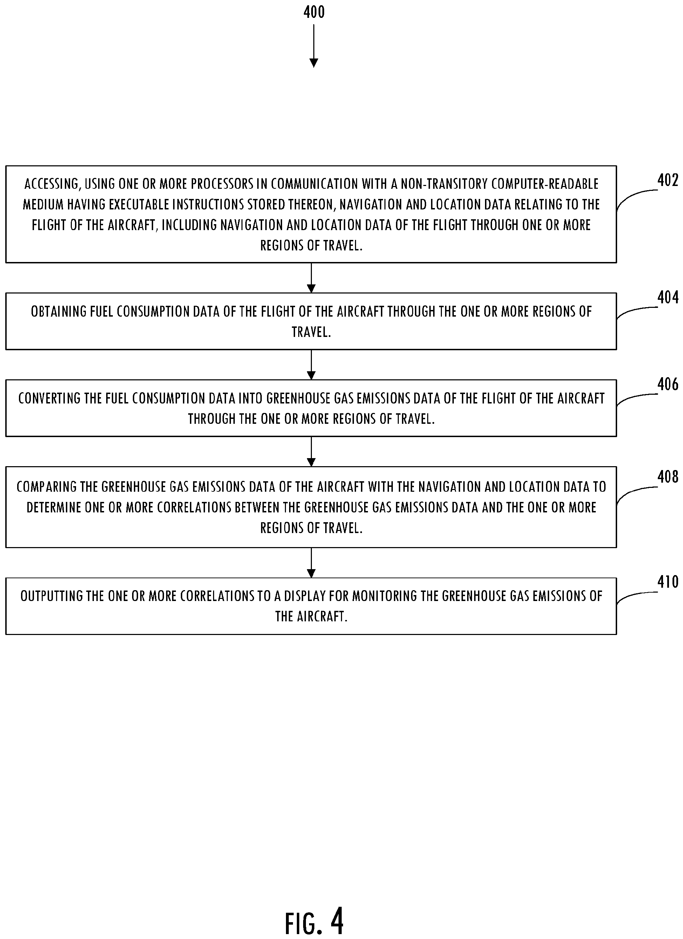

FIG. 4 illustrates a flow chart of an example method 400 for monitoring and predicting greenhouse gas emissions for a flight of an aircraft. As shown at block 402, the method includes accessing, using one or more processors in communication with a non-transitory computer-readable medium having executable instructions stored thereon, navigation and location data relating to the flight of the aircraft, including navigation and location data of the flight through one or more regions of travel. As shown at block 404, the method includes obtaining fuel consumption data of the flight of the aircraft through the one or more regions of travel.

As shown at block 406, the method 400 includes converting the fuel consumption data into greenhouse gas emissions data of the flight of the aircraft through the one or more regions of travel. As shown at block 408, the method includes comparing the greenhouse gas emissions data of the aircraft with the navigation and location data to determine correlations between the greenhouse gas emissions data and the one or more regions of travel. As shown at block 410, the method includes outputting the one or more correlations to a display for monitoring the greenhouse gas emissions of the aircraft.

According to examples of the present disclosure, the system 200 for monitoring and predicting greenhouse gas emissions for a flight of an aircraft is implemented by various means. Means for implementing the system includes hardware, alone or under direction of one or more computer programs from a computer-readable storage medium. In some examples, one or more apparatuses are configured to function as or otherwise implement the system shown and described herein. In examples involving more than one apparatus, the respective apparatuses are connected to or otherwise in communication with one another in a number of different manners, such as directly or indirectly via a wired or wireless network or the like.

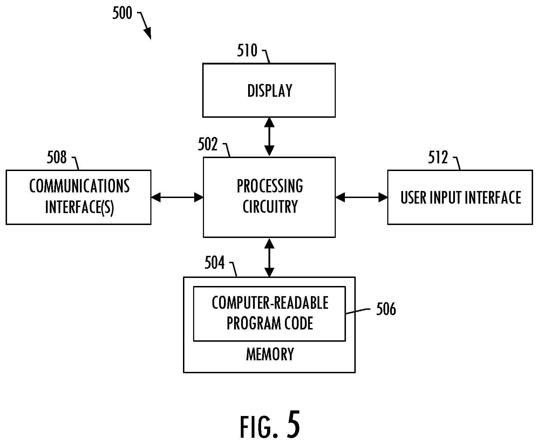

FIG. 5 illustrates an apparatus 500 capable of implementing the system 200 of FIG. 2A for monitoring and predicting greenhouse gas emissions for a flight of an aircraft. The apparatus 500 is an example device that is used to implement the methods and functions described above with respect to the system for monitoring and predicting greenhouse gas emissions for a flight of the aircraft. Generally, an exemplary apparatus of the present disclosure comprises, includes, or is embodied in one or more fixed or portable electronic devices. Examples of suitable electronic devices include a microcontroller, controller, smartphone, tablet computer, laptop computer, desktop computer, workstation computer, server computer or the like. The apparatus includes one or more of each of a number of components such as, for example, processing circuitry 502 (e.g., processor unit or computer processor) connected to a memory 504 (e.g., storage device).

The processing circuitry 502 is composed of one or more processors alone or in combination with one or more memories. The processing circuitry is generally any piece of computer hardware that is capable of processing information such as, for example, data, computer programs and/or other suitable electronic information. The processing circuitry is composed of a collection of electronic circuits some of which is packaged as an integrated circuit or multiple interconnected integrated circuits (an integrated circuit at times more commonly referred to as a "chip"). The processing circuitry is configured to execute computer programs, which are stored onboard the processing circuitry or otherwise stored in the memory 504 (of the same or another apparatus).

The processing circuitry 502 includes a number of processors, a multi-core processor or some other type of processor, depending on the particular implementation. Further, the processing circuitry is implemented using a number of heterogeneous processor systems in which a main processor is present with one or more secondary processors on a single chip. In other examples, the processing circuitry is a symmetric multi-processor system containing multiple processors of the same type. In yet other examples, the processing circuitry is embodied as or otherwise include one or more ASICs, FPGAs or the like. Thus, although the processing circuitry is capable of executing a computer program to perform one or more functions, the processing circuitry of various examples is capable of performing one or more functions without the aid of a computer program. In either instance, the processing circuitry is appropriately programmed to perform functions or operations according to examples of the present disclosure.

The memory 504 is generally any piece of computer hardware that is capable of storing information such as, for example, data, computer programs (e.g., computer-readable program code 506) and/or other suitable information either on a temporary basis and/or a permanent basis. The memory includes volatile and/or non-volatile memory, and is fixed or removable. Examples of suitable memory include random access memory (RAM), read-only memory (ROM), a hard drive, a flash memory, a thumb drive, a removable computer diskette, an optical disk, a magnetic tape or some combination of the above. Optical disks include compact disk - read only memory (CD-ROM), compact disk - read/write (CD-R/W), DVD or the like. In various instances, the memory is referred to as a computer-readable storage medium. The computer-readable storage medium is a non-transitory device capable of storing information, and is distinguishable from computer-readable transmission media such as electronic transitory signals capable of carrying information from one location to another. Computer-readable medium as described herein generally refer to a computer-readable storage medium or computer-readable transmission medium.

In addition to the memory 504, the processing circuitry 502 is also connected to one or more interfaces for displaying, transmitting and/or receiving information. The interfaces include a communications interface 508 (e.g., communications unit) and/or one or more user interfaces. The communications interface is configured to transmit and/or receive information, such as to and/or from other apparatus(es), network(s) or the like. The communications interface is configured to transmit and/or receive information by physical (wired) and/or wireless communications links. Examples of suitable communication interfaces include a network interface controller (NIC), wireless NIC (WNIC) or the like.

The user interfaces include a display 510 and/or one or more user input interfaces 512 (e.g., input/output unit). The display is configured to present or otherwise display information to a user, suitable examples of which include a liquid crystal display (LCD), light-emitting diode display (LED), plasma display panel (PDP) or the like. The user input interfaces are wired or wireless, and are configured to receive information from a user into the apparatus, such as for processing, storage and/or display. Suitable examples of user input interfaces include a microphone, image or video capture device, keyboard or keypad, joystick, touch-sensitive surface (separate from or integrated into a touchscreen), biometric sensor or the like. The user interfaces further include one or more interfaces for communicating with peripherals such as printers, scanners or the like.

As indicated above, program code instructions are stored in memory, and executed by processing circuitry that is thereby programmed, to implement functions of the systems, subsystems, tools and their respective elements described herein. As will be appreciated, any suitable program code instructions are loaded onto a computer or other programmable apparatus from a computer-readable storage medium to produce a particular machine, such that the particular machine becomes a means for implementing the functions specified herein. These program code instructions are also stored in a computer-readable storage medium that direct a computer, a processing circuitry or other programmable apparatus to function in a particular manner to thereby generate a particular machine or particular article of manufacture. The instructions stored in the computer-readable storage medium produce an article of manufacture, where the article of manufacture becomes a means for implementing functions described herein. The program code instructions are retrieved from a computer-readable storage medium and loaded into a computer, processing circuitry or other programmable apparatus to configure the computer, processing circuitry or other programmable apparatus to execute operations to be performed on or by the computer, processing circuitry or other programmable apparatus.

Retrieval, loading and execution of the program code instructions are performed sequentially such that one instruction is retrieved, loaded and executed at a time. In some examples, retrieval, loading and/or execution are performed in parallel such that multiple instructions are retrieved, loaded, and/or executed together. Execution of the program code instructions produce a computer-implemented process such that the instructions executed by the computer, processing circuitry or other programmable apparatus provide operations for implementing functions described herein.

Execution of instructions by a processing circuitry, or storage of instructions in a computer-readable storage medium, supports combinations of operations for performing the specified functions. In this manner, the apparatus 500 includes the processing circuitry 502 and the computer-readable storage medium or memory 504 coupled to the processing circuitry, where the processing circuitry is configured to execute computer-readable program code 506 stored in the memory. It will also be understood that one or more functions, and combinations of functions, are implemented by special purpose hardware-based computer systems and/or processing circuitry which perform the specified functions, or combinations of special purpose hardware and program code instructions.

Many modifications and other examples will come to mind to one skilled in the art to which these disclosed examples pertain having the benefit of the teachings presented in the foregoing descriptions and the associated drawings. Therefore, it is to be understood that examples are not to be limited to the specific examples disclosed and that modifications and other examples are intended to be included within the scope of the claims. Moreover, although the foregoing descriptions and the associated drawings describe examples in the context of certain example combinations of elements and/or functions, it should be appreciated that different combinations of elements and/or functions are provided by alternative examples without departing from the scope of the claims. In this regard, for example, different combinations of elements and/or functions than those explicitly described above are also contemplated within the scope of the claims. Although specific terms are employed herein, they are used in a generic and descriptive sense only and not for purposes of limitation.

It should be understood that although the terms first, second, etc. are used herein to describe various steps or calculations, these steps or calculations should not be limited by these terms. These terms are only used to distinguish one operation or calculation from another. For example, a first calculation is termed a second calculation, and, similarly, a second step is termed a first step, without departing from the scope of this disclosure. As used herein, the term "and/or" and the "/" symbol includes any and all combinations of one or more of the associated listed items.

As used herein, the singular forms "a", "an" and "the" are intended to include the plural forms as well, unless the context clearly indicates otherwise. It will be further understood that the terms "comprises", "comprising", "includes", and/or "including", when used herein, specify the presence of stated features, integers, steps, operations, elements, and/or components, but do not preclude the presence or addition of one or more other features, integers, steps, operations, elements, components, and/or groups thereof. Therefore, the terminology used herein is for the purpose of describing particular examples only and is not intended to be limiting.

## Claims

1. A method (400) for monitoring greenhouse gas emissions for a flight of an aircraft (102), the method (400) comprising:
accessing (402), using one or more processors (201) in communication with a non-transitory computer-readable medium having executable instructions (506) stored thereon, navigation and location data relating to the flight of the aircraft (102), including navigation and location data of the flight through one or more regions of travel (105, 107);
obtaining (404) fuel consumption data of the flight of the aircraft (102) through the one or more regions of travel (105, 107);
converting (406) the fuel consumption data into greenhouse gas emissions data of the flight of the aircraft (102) through the one or more regions of travel (105, 107);
comparing (408) the greenhouse gas emissions data of the aircraft (102) with the navigation and location data to determine one or more correlations between the greenhouse gas emissions data and the one or more regions of travel (105, 107); and
outputting (410) the one or more correlations to a display (210) for monitoring the greenhouse gas emissions of the aircraft (102).

2. The method (400) of claim 1, wherein obtaining the fuel consumption data of the flight of the aircraft (102) includes calculating fuel consumption of the flight using mathematical models (207) based on any or all of location and navigation data, aircraft type and configuration, and weather data.

3. The method (400) of claim 2, wherein aircraft type and configuration includes data regarding any or all of aircraft model or class, aircraft weight, a number of seats onboard the aircraft, winglet parameters, engine type, and a load factor of the aircraft (102).

4. The method (400) of claim 2 or 3, wherein the weather data includes data regarding any or all of wind intensity and direction, temperature, and humidity of surroundings of the aircraft (102) during the flight.

5. The method (400) of any preceding claim, wherein obtaining the fuel consumption data of the flight of the aircraft (102) includes obtaining the fuel consumption data directly from the aircraft (102).

6. The method (400) of any preceding claim, wherein obtaining the fuel consumption data of the flight of the aircraft (102) includes obtaining flight parameter data of the flight; and
wherein converting the fuel consumption data into greenhouse gas emissions data includes applying a mathematical model (207) to one or both of the fuel consumption data and the flight parameter data to obtain the greenhouse gas emissions data.

7. The method (400) of claim 6, wherein the mathematical model (207) is a linear relationship function and the greenhouse gas emissions data comprises carbon dioxide emissions data.

8. The method (400) of claim 6 or 7, wherein the greenhouse gas emissions data comprises emissions data for greenhouse gasses other than carbon dioxide.

9. The method (400) of any of claims 6 to 8, comprising applying the mathematical model (207) to both the fuel consumption data and the flight parameter data, and the flight parameter data includes at least flight altitude data and climatic conditions surrounding the aircraft (102).

10. The method (400) of any preceding claim, wherein the one or more correlations include correlations characterizing quantities of greenhouse gas emissions of the aircraft (102) over a given area of the one or more regions of travel (105, 107).

11. A method (400) for monitoring and predicting greenhouse gas emissions for a flight of an aircraft (102), the method (400) comprising:
the method (400) for monitoring greenhouse gas emissions of any preceding claim;
building a machine learning model (235) for predicting greenhouse gas emissions for a current or future flight of the aircraft (102) using a machine learning algorithm trained with the one or more correlations between the greenhouse gas emissions data and the one or more regions of travel (105, 107), one or more correlations between the greenhouse gas emissions and weather data, and the navigation and location data;
predicting the greenhouse gas emissions of the aircraft (102) for the current or future flight of the aircraft (102) for the region of travel using the machine learning model (235) and based on the one or more correlations between the greenhouse gas emissions data and the one or more regions of travel (105, 107); and
outputting the prediction to the display (210) for use by a user to make decisions about altering the current or future flight of the aircraft (102).

12. A system for monitoring and predicting greenhouse gas emissions for a flight of an aircraft (102), the system comprising:
one or more computer processors (201) in communication with a non-transitory computer-readable medium having executable instructions (506) stored thereon, wherein, upon execution of the executable instructions (506), the one or more computer processors (201) perform the method of any preceding claim.

13. An aircraft (102) including the system of claim 12.

14. A computer program comprising compute program instructions that, when executed by one or more computer processors, cause the one or more computer processors to perform the method of any of claims 1 to 11.

15. A computer readable medium having stored thereon the computer program of claims 14.
